# FASCICULE DE BREVET EUROPEEN

(11) **EP 1 711 552 B1**
(45) Date de publication et mention de la délivrance du brevet: **24.11.2010**
(21) Numéro de dépôt: 05717516.8
(22) Date de dépôt: 31.01.2005
(51) Int. Cl.: C08J 3/24, C08J 3/075, A61L 27/52, A61L 27/26

(54) **GEL RETICULE BIOCOMPATIBLE**
BIOKOMPATIBLES VERNETZTES GEL
BIOCOMPATIBLE CROSSLINKED GEL

(30) Priorité: 03.02.2004 FR 0400987
(43) Date de publication de la demande: 18.10.2006
(73) Titulaire: ANTEIS S.A., 1228 Plan-Les-Ouates, Genève (CH)
(72) Inventeur: HERMITTE, Laurence, F-13080 Luynes (FR); BENOIT, Olivier, 74330 Epagny (FR)
(74) Mandataire: Brema-Loyer
(86) Numéro de dépôt international: PCT/FR2005/000197
(87) Numéro de publication internationale: WO 2005/085329

(56) Documents cités:
- EP-A- 0 161 887
- WO-A-02/06350
- WO-A-96/33751
- WO-A-97/04012

## Description

La présente invention concerne un procédé de fabrication d'un gel réticulé biocompatible, ledit gel et l'utilisation dudit gel pour constituer une matrice comportant au moins un principe actif dispersé ou pour séparer, remplacer ou combler un tissu biologique ou augmenter le volume dudit tissu ou encore supplémenter ou remplacer un fluide biologique.

L'augmentation du volume tissulaire peut être souhaitée à la fois dans le cas d'applications thérapeutiques et dans un but cosmétique. Elle peut être effectuée par introduction d'une solution viscoélastique à base de produits permanents ou biodégradables dans les tissus biologiques.

L'injection de solutions viscoélastiques à base de produits permanents ou biodégradables est également envisagée pour remplacer un fluide biologique.

Par exemple, elle est utilisée pour remplacer le liquide synovial naturel qui, chez les patients arthrosiques, ne peut plus assurer ses fonctions chondroprotectrices, de lubrification et d'absorption des chocs compte tenu d'une réduction de la quantité et de la masse moléculaire des glycosaminoglycanes constitutifs. Mais cette solution viscoélastique, lorsqu'elle est constituée à base de produits biodégradables, est rapidement éliminée de la poche synoviale.

Dans le cas d'autres applications thérapeutiques, ce type de solution viscoélastique est utilisé pour certains tissus qui nécessitent d'être élargis pour assurer leur fonction ; il s'agit par exemple des cordes vocales, de l'oesophage, du sphincter ou de l'urètre.

Dans le cas des applications cosmétiques, ce type de solution viscoélastique est utilisé par exemple, pour le comblement des rides, le masquage des cicatrices, ou l'augmentation du volume des lèvres. L'injection de ces solutions viscoélastiques est une méthode simple, non invasive, moins risquée et moins onéreuse que la chirurgie esthétique.

L'utilisation de solution viscoélastique à base de produits permanents présente l'avantage d'une longue rémanence dans les tissus où la solution viscoélastique est injectée.

L'injection de silicone a très longtemps été utilisée. Cependant, compte tenu des effets indésirables à long terme de cette méthode, qui se caractérisent par l'apparition de nodules et d'ulcère de la peau, cette pratique est peu à peu abandonnée.

L'injection de microparticules solides en suspension permet également une augmentation du volume tissulaire permanente. Le brevet US 5,344,452 décrit l'utilisation d'un solide pulvérulent, constitué de petites particules, de diamètre compris entre 10µm et 200µm, et ayant une surface très lisse. Artecoll® et Arteplast®, produits commercialisés, sont constitués d'une suspension de microsphères de polyméthacrylate dans une solution de collagène. Le brevet EP 1 091 775 propose une suspension de fragments d'hydrogel de méthacrylate dans une solution de hyaluronate. Les particules de silicone, céramiques, de carbone ou métalliques (brevets US 5,451,406, US 5,792,478 et demande US2002151466), et les fragments de polytétrafluoroéthylène, de verre ou de polymères synthétiques (demande US2002025340) ont également été utilisées mais les résultats sont décevants. En effet, des réactions secondaires, issues de la dégradation biologique de la solution de mise en suspension biodégradable et de la migration des fragments résiduels qui peuvent induire une réaction inflammatoire, peuvent survenir. En outre, l'injection de particules au travers d'une aiguille fine peut être difficile si les particules ont une diamètre trop important ou une forme irrégulière qui peut provoquer l'agglutination des particules entre elles. De plus, l'injection de particules fragiles peut endommager leur structure ce qui aboutit à l'injection de particules trop fines qui n'adhèrent pas aux cellules environnantes mais migrent vers d'autres tissus, ou sont rapidement digérées par les macrophages et les autres constituants du système lymphatique.

D'une manière générale, le caractère permanent de ces produits induit des inconvénients majeurs (US 6 436 424) qui sont notamment le risque d'activation des macrophages, la migration des fragments synthétiques constitutifs du produit pouvant provoquer une réaction inflammatoire pouvant même aboutir à l'apparition de granulomes. Le traitement de ces granulomes nécessite alors soit un traitement thérapeutique par injection de stéroïdes soit un traitement chirurgical par excision, ces traitements pouvant avoir des conséquences lourdes sur la santé du patient ou sa qualité de vie. Par conséquent, les effets secondaires des produits permanents sont si néfastes qu'ils découragent l'utilisation de ces produits dans un but purement esthétique. De plus, l'injection de solution viscoélastique à base de produits permanents ne permet pas de retouche si nécessaire.

Parmi les matériaux biologiquement dégradables, il existe des suspensions de collagène ou d'acide hyaluronique réticulé.

Collagen Corporation a développé une préparation à base de collagène réticulé avec du glutaraldéhyde décrite dans le brevet US 4 582 640. Cependant, ce produit est dégradé rapidement au sein du tissu où il est injecté, par les macrophages, ou par action enzymatique ou chimique et est ensuite éliminé du tissu par le système lymphatique. Le brevet US 5 137 875 propose l'utilisation de suspensions ou solutions aqueuses de collagène contenant de l'acide hyaluronique, mais ce produit ne peut constituer un traitement à long terme car il est également rapidement digéré puis éliminé par le système lymphatique. Des traitements répétés sont donc nécessaires ce qui engendre un coût considérable et diminue la qualité de vie du patient.

Le brevet EP 0 466 300 propose l'injection d'un gel viscoélastique biphasique composé d'une matrice dispersée dans une phase liquide, les deux phases étant composées par du hyalan, hyaluronate de haute masse moléculaire d'origine animale, réticulé et extrait. L'utilisation d'un polymère de haute masse moléculaire permet une plus grande rémanence du gel viscoélastique biodégradable dans le tissu. Cette technologie a donné lieu à plusieurs produits sur le marché tels que le Hylaform®, pour le comblement des dépressions de la matrice intercellulaire du tissu conjonctif, ou le Synvisc®, produit de viscosupplémentation pour le traitement de l'arthrose.

Parmi les produits biodégradables biphasiques, peuvent également être cités Restylane®, Macrolane®, Perlant®, ou Durolane®, autres compositions biphasiques constituées d'une phase fluide (hyaluronate non réticulé) et d'une phase composée d'acide hyaluronique très réticulé. Destinés à l'augmentation du volume tissulaire (visage, seins) ou au traitement arthrosique, ces produits sont basés sur la technologie NASHA détenue par Q-Med. Il a été également observé que, dans certains cas, l'utilisation de produits biphasiques pouvait induire des réactions inflammatoires voire engendrer l'apparition de granulomes (Laeschke K. Biocompatibility of microparticles into soft tissues fillers. Congress of Aesthetic Medicine and Dermatologie Surgery, Paris, 2003) même si ces réactions sont moins observées qu'en présence d'un gel à base de polymères synthétiques. De plus, la phase fluide est très rapidement éliminée, ce qui engendre une perte de matière correspondant au volume de cette phase fluide. Par conséquent, lorsqu'une augmentation du volume tissulaire est recherchée, de nombreuses retouches sont nécessaires après la première injection, ce qui diminue la qualité de vie de l'utilisateur.

Enfin, plusieurs gels viscoélastiques monophasiques ont été proposés soit pour homogénéiser le taux de réticulation au sein du gel (demande de brevet US 20030148995), soit pour contrôler la biodégradabilité du gel (US 4 963 666), soit pour contrôler les propriétés viscoélastiques du gel (US 5 827 937). Une forte réticulation des polymères permet une plus grande rémanence dans le tissu du gel viscoélastique biodégradable. Cependant l'injection du gel comportant un tel polymère fortement réticulé est plus difficile. De plus, l'injection d'un tel gel fragilise mécaniquement les sites non réticulés du polymère qui deviennent plus vulnérables à des attaques biochimiques et enzymatiques, ce qui favorise une dégradation rapide du gel.

L'invention a pour but de proposer un gel réticulé biocompatible qui évite les inconvénients précités, qui présente les avantages simultanés de mise en oeuvre facile dans son utilisation clinique et de durée de vie telle que le gel réticulé biocompatible disparaisse lorsque sa fonction n'est plus souhaitée, mais suffisante pour limiter le nombre d'administrations par actes médicaux ou chirurgicaux.

A cet effet, l'invention a pour objet un procédé de fabrication d'un gel réticulé biocompatible comportant les étapes suivantes :
une étape d'amorçage de la réticulation d'une quantité déterminée d'au moins un polymère biocompatible en solution par l'ajout d'une quantité d'agent réticulant,
une étape de réaction de réticulation de ladite quantité de polymère,
une étape d'ajout d'une quantité supplémentaire de polymère de masse moléculaire supérieure à 500 000 Da en solution avec dilution du mélange réactionnel de manière à diminuer la concentration globale du polymère en solution, en poursuivant l'étape de réticulation, et
une étape d'arrêt de la réaction de réticulation par élimination de l'agent réticulant.

L'étape d'ajout d'une quantité supplémentaire de polymère permet l'apport de sites réactionnels nouveaux.

Ce procédé permet d'obtenir un gel réticulé biocompatible présentant simultanément les caractéristiques d'être monophasique, polydensifié, cohésif, injectable et à longue rémanence.

Par cohésif, on entend une tendance du gel à se regrouper et non à se répandre ou se fragmenter. Le caractère cohésif contribue donc à l'obtention d'une compatibilité élevée et d'une longue rémanence *in vivo* du gel.

Par polydensifié, on entend une variation du degré de réticulation au sein même du gel. Le caractère polydensifié du gel permet à la composition de cumuler les avantages d'injectabilité au travers d'une aiguille de faible diamètre et de longue rémanence *in vivo* du gel.

Le caractère monophasique permet de réduire les risques de réactions inflammatoires et d'apparition de granulomes.

L'effet de longue rémanence du gel permet d'espacer les actes médicaux et donc d'améliorer la qualité de vie des patients.

Un tel gel monophasique polydensifié cohésif obtenu suivant le procédé de la présente invention se caractérise par une injectabilité facilitée et unie rémanence *in vivo* supérieure à celle d'un gel monophasique de même composition, dont le taux de réticulation est homogène au sein du gel.

Selon un mode de réalisation particulier de l'invention, l'étape d'amorçage de la réticulation est réalisée en milieu basique.

Selon un autre mode de réalisation de l'invention, l'étape d'amorçage de la réticulation est réalisée en milieu acide.

Préférentiellement, l'étape d'arrêt de la réticulation est réalisée par dialyse. La dialyse assure l'arrêt final de la réaction. Elle élimine l'agent réticulant et les petites chaînes de polymère n'ayant pas réagi.

Avantageusement, les polymères sont d'origine naturelle. L'utilisation de polymères d'origine naturelle permet une meilleure biocompatibilité c'est-à-dire qu'une telle utilisation engendre moins de risques de réaction inflammatoire.

Préférentiellement, les polymères d'origine naturelle sont des composés choisis dans le groupe constitué par : l'acide hyaluronique, la chondroïtine sulfate, le kératane, le kératane sulfate, l'héparine, l'héparane sulfate, la cellulose et ses dérivés, les alginates, le xanthane, la carraghénine, les protéines ou les acides nucléiques.

De manière encore plus avantageuse, au moins un polymère d'origine naturelle est un polymère non naturellement présent chez l'être humain choisi dans le groupe constitué par : la cellulose et ses dérivés, les alginates, le xanthane, la carraghénine, polymère qui est réticulé avec au moins un polymère naturellement présent chez l'être humain choisi dans le groupe constitué par : l'acide hyaluronique, la chondroïtine sulfate, le kératane, le kératane sulfate, l'héparine, l'héparane sulfate, les protéines ou les acides nucléiques.

Avantageusement, l'agent réticulant est une molécule bi- ou poly-fonctionnelle sélectionnée parmi les composants du groupe constitué par les époxydes, les épihalohydrines et la divinylsulfone.

L'invention a également pour objet un gel préparé par le procédé susmentionné.

De manière préférentielle, le gel constitue une matrice comportant au moins un principe actif dispersé. Le gel sera alors utilisé comme vecteur permettant une libération progressive dudit principe actif au sein du liquide ou du tissu biologique où il a été injecté.

Enfin, l'invention a pour objet l'utilisation de ce gel pour séparer, remplacer ou combler un tissu biologique ou augmenter le volume dudit tissu ou encore supplémenter ou remplacer un fluide biologique.

L'invention sera mieux comprise, et d'autres buts, détails, caractéristiques et avantages de celle-ci apparaîtront plus clairement au cours de la description explicative détaillée qui va suivre, d'un mode de réalisation de l'invention donné à titre d'exemple purement illustratif et non limitatif.

Le procédé de fabrication du gel réticulé biocompatible se caractérise par les étapes successives d'amorçage de la réticulation d'une quantité déterminée d'au moins un polymère biocompatible en solution, de réaction de réticulation de ladite quantité de polymère, d'ajout d'une quantité supplémentaire de polymère de masse moléculaire supérieure à 500 000 Da en solution avec dilution du mélange réactionnel de manière à diminuer la concentration globale du polymère en solution, en poursuivant l'étape de réticulation, et d'arrêt de la réaction de réticulation par élimination de l'agent réticulant.

L'étape d'amorçage de la réticulation est réalisée par l'ajout d'une quantité d'agent réticulant qui est une molécule bi- ou poly-fonctionnelle sélectionnée parmi les composants du groupe constitué par les époxydes, les épihalohydrines et la divinylsulfone. Les époxydes préférés sont les composants choisis dans le groupe constitué par : le 1,4 butanediol diglycidyl éther (aussi appelé 1,4-bis (2,3-époxypropoxy)butane), le 1-(2,3- époxypropyl) 2,3- époxy cyclohexane et le 1,2-éthanediol diglycidyl éther.

Selon un mode de réalisation particulier de l'invention, l'étape d'amorçage de la réticulation est réalisée en milieu basique. La réaction de réticulation effectuée en milieu basique se traduit par la formation de liaisons éther qui sont très solides. La réticulation par éthérification permet une plus longue rémanence *in vivo*.

Selon un autre mode de réalisation de l'invention, l'étape d'amorçage de la réticulation est réalisée en milieu acide. La réaction de réticulation effectuée en milieu acide se traduit par la formation de liaisons ester qui sont plus labiles que les liaisons éther précédemment citées. Cependant une plus grande labilité des pontages peut présenter certains avantages. Notamment, un tel gel utilisé comme matrice comportant un principe actif dispersé permet une autre cinétique de libération dudit principe actif plus adaptée à certaines applications.

La réaction de réticulation est la réaction qui assure le pontage des chaînes de chaque polymère entre eux. Elle peut être quantifiée par la détermination du taux de réticulation.

Le taux de réticulation est défini comme le rapport entre le nombre de moles d'agent réticulant assurant le pontage des chaînes de chaque polymère et le nombre de moles de motifs du polymère.

La réticulation s'effectue dans une plage de températures préférentielles de 25°C à 60°C.

La réticulation peut s'effectuer sur un même polymère ou sur un mélange de polymères.

Les polymères engagés dans la réaction de réticulation peuvent être synthétiques mais sont avantageusement d'origine naturelle. L'utilisation de polymères d'origine naturelle permet une meilleure biocompatibilité, c'est-à-dire qu'une telle utilisation engendre moins de risques de réaction inflammatoire.

Préférentiellement, on utilise les polymères d'origine naturelle susmentionnés.

Il est cependant bien évident que l'invention ne se limite pas aux polymères susmentionnées mais peut utiliser des polymères de nature et de taille différente.

L'étape d'ajout d'une quantité supplémentaire de polymère s'accompagne d'une dilution du milieu réactionnel de sorte que la concentration totale en polymère de la solution diminue.

Dans ces conditions, les chaînes de polymère apportant des sites de réticulation nouveaux vont réagir avec l'agent de réticulation résiduel et/ou ajouté en faible quantité, en se fixant sur le gel réticulé initial et entre elles avec un plus faible taux de réticulation puisque la quantité d'agent de réticulation a diminué. Le nombre de pontages sur les chaînes de gel formé dans la première étape de réticulation est supérieur au nombre de pontages entre celui-ci et les chaînes ajoutées et au nombre de pontages entre les chaînes ajoutées. Le degré de réticulation varie donc au sein du gel final qui est constitué de noyaux fortement réticulés (par exemple avec un taux de réticulation de 25%) reliés entre eux par un gel de moins en moins réticulé (dont le taux de réticulation diminue progressivement et peut atteindre 1%). Cette particularité lui confère propriétés viscoélastiques exceptionnelles qui permettent au gel, tout en présentant un taux de réticulation important et donc une longue rémanence *in vivo,* d'être cohésif (un seul et même gel) et injectable par toute sorte de dispositifs médicaux et notamment les aiguilles fines.

L'ajout de polymères supplémentaires s'effectue à tout niveau d'avancement de la réaction de réticulation initiale, avantageusement à 75% de la réaction de réticulation initiale. Cette étape peut être effectuée par apport de polymère de façon continue ou discontinue.

Les polymères supplémentaires doivent présenter une masse moléculaire supérieure à 500 000 Da. Ils peuvent également être synthétiques ou naturels. Ils peuvent être ajoutés sous forme de mélange de polymères. Ils peuvent être de nature ou de taille identique ou différente de ceux utilisés dans l'étape de réticulation initiale. De manière avantageuse, les polymères supplémentaires ajoutés sont constitués de plus longues chaînes que les polymères présents initialement. Ceci confère au gel une amélioration de sa structure mécanique externe, les longues chaînes étant plus difficilement dégradées que les courtes.

Ce procédé permet par conséquent d'obtenir un gel réticulé biocompatible présentant simultanément les caractéristiques d'être monophasique, polydensifié, cohésif, injectable et à longue rémanence.

Selon un certain mode de réalisation de l'invention, une quantité supplémentaire d'agent réticulant est ajoutée lors de l'étape d'ajout d'une quantité supplémentaire de polymère. Cet agent de réticulation pourra être de nature identique ou différente à celle de celui utilisé lors de la réaction d'amorçage de la réaction. Il est préférentiellement choisi parmi les composants du groupe susmentionné. La quantité ajoutée est nettement inférieure à la quantité ajoutée pour la réticulation initiale.

L'étape d'arrêt de la réaction de réticulation assure l'arrêt final de la réaction. Elle est par exemple réalisée par dialyse qui permet d'éliminer l'agent réticulant et les petites chaînes de polymère n'ayant pas réagi. En effet l'injection d'un gel comportant un tel agent induit des réactions inflammatoires car ces agents sont des composants chimiques difficilement assimilables et très réactifs.

De manière préférentielle, le gel constitue une matrice comportant au moins un principe actif dispersé. Le gel est alors utilisé comme vecteur permettant une libération progressive dudit principe actif au sein du liquide ou du tissu biologique où il a été injecté. Le principe actif est un agent pharmacologiquement actif pouvant être par exemple un agent anti-oxydant. Le principe actif peut également être de nature différente. Un mélange de principes actifs de nature différente peut également être dispersé dans le gel.

Ce gel est préférablement injecté.

Enfin, le gel est avantageusement utilisé pour séparer, remplacer ou combler un tissu biologique ou augmenter le volume dudit tissu par exemple dans le cas d'applications thérapeutiques (augmentation du volume des cordes vocales, de l'oesophage, du sphincter, de l'urètre ou d'autres organes) ou dans un but cosmétique pour le comblement des rides, le masquage des cicatrices, ou l'augmentation du volume des lèvres.

Il peut également supplémenter ou remplacer un fluide biologique, par exemple le liquide synovial naturel.

Des exemples sont proposés afin d'illustrer l'invention mais ne sont nullement limitatifs de l'invention.

### Exemple 1 (comparatif)

10 g d'acide hyaluronique (MM=2x10⁶Da) sont dilués dans 100 ml d'une solution de NaOH à 1%. L'acide hyaluronique est hydraté par cette étape préalable à la réticulation.

Le tout est homogénéisé dans un mélangeur jusqu'à ce qu'une solution transparente soit obtenue.

La réaction de réticulation est alors amorcée par ajout de 470 µl de 1,4-butanediol diglycidyl éther (BDDE) à la solution et le tout est mélangé pendant 15h à 25°C, dans une atmosphère privée d'oxygène.

Le pH est réajusté au pH physiologique à l'aide d'HCl 1M. Le volume est ajusté à 400 ml à l'aide d'une solution tamponnée à pH=7.

Le gel obtenu est ensuite dialysé pendant 24 h (cellulose régénérée, limite de séparation : MM=60 kDa) contre une solution tamponnée à pH=7 (Gel I).

Ce gel a une teneur en acide hyaluronique totale de 2,5% en masse.

### Exemple 2 (comparatif)

Le gel est ici réalisé de la même manière que dans l'exemple 1 sauf qu'un quantité plus importante d'agent réticulant est ajoutée.

10 g d'acide hyaluronique (MM=2x10⁶Da) sont dilués dans 100 ml d'une solution de NaOH à 1 %.

Le tout est homogénéisé dans un mélangeur jusqu'à ce qu'une solution transparente soit obtenue.

760 µl de 1,4-butanediol diglycidyl éther (BDDE) sont ensuite ajoutés à la solution et le tout est mélangé pendant 15 h à 25°C, dans une atmosphère privée d'oxygène.

Le pH est réajusté au pH physiologique à l'aide d'HCl 1M.

Le volume est ajusté à 400 ml à l'aide d'une solution tamponnée à pH=7.

Le gel obtenu est ensuite dialysé pendant 24 h (cellulose régénérée, limite de séparation : MM=60 kDa) contre une solution tamponnée à pH=7 (Gel II).

Ce gel a une teneur en acide hyaluronique totale de 2,5% en masse.

### Exemple 3 comparatif)

Le gel est ici réalisé de la même manière que dans les exemples 1 ou 2 sauf qu'un quantité encore plus importante d'agent réticulant est ajoutée.

10 g d'acide hyaluronique (MM=2x10⁶ Da) sont dilués dans 100 ml d'une solution de NaOH à 1%.

Le tout est homogénéisé dans un mélangeur jusqu'à ce qu'une solution transparente soit obtenue.

950 µl de 1,4-butanediol diglycidyl éther (BDDE) sont ensuite ajoutés à la solution et le tout est mélangé pendant 15 h à 25°C, dans une atmosphère privée d'oxygène.

Le pH est réajusté au pH physiologique à l'aide d'HCl 1M.

Le volume est ajusté à 400 ml à l'aide d'une solution tamponnée à pH=7 et l'ensemble est homogénéisé.

Le gel obtenu est ensuite dialysé pendant 24 h (cellulose régénérée, limite de séparation : MM=60 kDa) contre une solution tamponnée à pH=7 (Gel III).

Ce gel a une teneur en acide hyaluronique totale de 2,5% en masse.

### Exemple 4 (selon l'invention)

10 g d'acide hyaluronique (MM=2x10⁶Da) sont dilués dans 100 ml d'une solution de NaOH à 1%.

Le tout est homogénéisé dans un mélangeur jusqu'à ce qu'une solution transparente soit obtenue.

La réaction de réticulation est amorcée par l'ajout de 950 µl de 1,4-butanediol diglycidyl éther (BDDE) sont ensuite ajoutés à la solution et le tout est mélangé pendant 9 h à 25°C, dans une atmosphère privée d'oxygène.

Puis, un polymère supplémentaire est ajouté tout en ajustant le volume à 300 ml à l'aide d'une solution d'acide hyaluronique 0,5% pH=11 (MM=2x10⁶Da).

La réaction continue encore 6 h. Le pH est réajusté au pH physiologique à l'aide d'HCl 1M et le volume ajusté à 400 ml.

L'ensemble est homogénéisé.

Afin d'arrêter définitivement la réaction de réticulation, le gel obtenu est ensuite dialysé pendant 24 h (cellulose régénérée, limite de séparation : MM=60 kDa) contre une solution tamponnée à pH=7 (Gel IV).

Seul ce dernier gel est réalisé selon l'invention, les trois autres types de gel étant réalisés selon l'état de la technique, c'est-à-dire avec une réticulation uniforme.

Ce gel a une teneur en acide hyaluronique totale de 2,75% en masse.

Une étude rhéologique a été réalisée sur les gels présentés dans les exemples 1 à 4.

Cette étude consiste en la mesure de la force limite d'extrusion (F) d'un gel c'est-à-dire la force à partir de laquelle le gel peut être extrudé.

Pour cela, le gel est contenu dans un cylindre en acier inoxydable de 2,5 cm de diamètre et extrudé au travers d'un pore de 0,2mm de diamètre.

Les résultats obtenus sont présentés par le tableau suivant :

| Type de gel | F (N/mm²) σ=0,15N/mm² |
|---|---|
| I | 3,56 |
| II | 5,85 |
| III | 7,40 |
| IV | 6,12 |

| | |
|---|---|
| σ: écart type | |

Les gels I, II et III représentent des gels dont le taux de réticulation est constant dans le gel. Seul le gel IV présente un gel dont le taux de réticulation est variable.

Cette méthode met en évidence tout d'abord qu'un ajout croissant d'agent réticulant (entre les gels de type I à III) se traduit par une force limite d'extrusion également plus importante, c'est-à-dire que la force à appliquer pour extruder un gel à taux de réticulation croissant augmente pour les gels présentant un taux de réticulation homogène.

Le gel de type IV (gel selon l'invention) à 2,75% en masse d'acide hyaluronique s'injecte quasi aussi facilement qu'un gel à 2,5% en masse d'acide hyaluronique de taux de réticulation plus faible et homogène (gel de type II) et plus facilement (avec une force F 15% plus faible) qu'un gel à 2,5% en masse d'acide hyaluronique dont le taux de réticulation est identique mais homogène (gel de type III).

Par conséquent, cet exemple prouve qu'un gel selon l'invention, dont le taux de réticulation est hétérogène, permet, à un taux de réticulation important, et donc de rémanence *in vivo* importante, d'être facilement extrudé par des dispositifs de type aiguille fine.

## Revendications

1. Procédé de fabrication d'un gel réticulé biocompatible comportant : une étape d'amorçage de la réticulation d'une quantité déterminée d'au moins un polymère biocompatible en solution par l'ajout d'une quantité d'agent réticulant,
une étape de réaction de réticulation de ladite quantité de polymère,
une étape d'ajout d'une quantité supplémentaire de polymère de masse moléculaire supérieure à 500 000 Da en solution avec dilution du mélange réactionnel de manière à diminuer la concentration globale du polymère en solution, en poursuivant l'étape de réticulation, et
une étape d'arrêt de la réaction de réticulation par élimination de l'agent réticulant.

2. Procédé selon la revendication 1, **caractérisé en ce que** l'étape d'amorçage de la réticulation est réalisée en milieu basique.

3. Procédé selon la revendication 1, **caractérisé en ce que** l'étape d'amorçage de la réticulation est réalisée en milieu acide.,

4. Procédé selon l'une des revendications 1 à 3, **caractérisé en ce qu'**une quantité supplémentaire d'agent réticulant est ajoutée lors de l'étape d'ajout d'une quantité supplémentaire de polymère.

5. Procédé selon l'une des revendications 1 à 4, **caractérisé en ce que** l'étape d'arrêt de la réticulation est réalisée par dialyse.

6. Procédé selon l'une des revendications 1 à 5, **caractérisé en ce que** les polymères sont d'origine naturelle.

7. Procédé selon la revendication 6, **caractérisé en ce que** les polymères d'origine naturelle sont des composés choisis dans le groupe constitué pair: l'acide hyaluronique, la chondroïtine sulfate, le kératane, le kératane sulfate, l'héparine, l'héparane sulfate, la cellulose et ses dérivés, les alginates, le xanthane, la carraghénine, les protéines ou les acides nucléiques.

8. Procédé selon la revendication 6, **caractérisé en ce qu'**au moins un polymère d'origine naturelle est un polymère non naturellement présent chez l'être humain choisi dans le groupe constitué par : la cellulose et ses dérivés, les alginates, le xanthane, la carraghénine, polymère qui est réticulé avec au moins un polymère naturellement présent chez l'être humain choisi dans le groupe constitué par : l'acide hyaluronique, la chondroïtine sulfate, le kératane, le kératane sulfate, l'héparine, l'héparane sulfate, les protéines ou les acides nucléiques.

9. Procédé selon l'une des revendications 1 à 8, **caractérisé en ce que** l'agent réticulant est une molécule bi- ou poly-fonctionnelle sélectionnée parmi les composants du groupe constitué par les époxydes, les épihalohydrines et la divinylsulfone.

10. Gel préparé par le procédé selon l'une des revendications 1 à 9.

11. Gel selon la revendication 10, **caractérisé en ce qu'**il constitue un gel comportant au moins un principe actif dispersé.

12. Utilisation du gel selon la revendication 10 ou 11, pour séparer, remplacer ou combler un tissu biologique ou augmenter le volume dudit tissu ou encore supplémenter ou remplacer un fluide biologique.

## Claims

1. Method for producing a biocompatible crosslinked gel, comprising: a step of starting the crosslinking of a given quantity of at least one biocompatible polymer in solution by adding a quantity of crosslinking agent,
a step involving the crosslinking reaction of said quantity of polymer,
a step of adding an additional quantity of polymer having a molecular weight greater than 500,000 Da in solution with dilution of the reaction mixture so as to reduce the overall concentration of the polymer in solution, while continuing with the crosslinking step, and
a step of stopping the crosslinking reaction by removing the crosslinking agent.

2. Method according to claim 1, **characterised in that** the step of starting the crosslinking is carried out in a basic medium.

3. Method according to claim 1, **characterised in that** the step of starting the crosslinking is carried out in an acidic medium.

4. Method according to one of claims 1 to 3, **characterised in that** an additional quantity of crosslinking agent is added during the step of adding an additional quantity of polymer.

5. Method according to one of claims 1 to 4, **characterised in that** the step of stopping the crosslinking is carried out by dialysis.

6. Method according to one of claims 1 to 5, **characterised in that** the polymers are of natural origin.

7. Method according to claim 6, **characterised in that** the polymers of natural origin are compounds selected from the group consisting of:
hyaluronic acid, chondroitin sulphate, keratan, keratan sulphate, heparin, heparin sulphate, cellulose and derivatives thereof, alginates, xanthan, carrageenan, proteins or nucleic acids.

8. Method according to claim 6, **characterised in that** at least one polymer of natural origin is a polymer that is not naturally present in human beings, selected from the group consisting of: cellulose and derivatives thereof, alginates, xanthan, carrageenan, which polymer is crosslinked with at least one polymer that is naturally present in human beings, selected from the group consisting of: hyaluronic acid, chondroitin sulphate, keratan, keratan sulphate, heparin, heparin sulphate, proteins or nucleic acids.

9. Method according to one of claims 1 to 8, **characterised in that** the crosslinking agent is a bifunctional or polyfunctional molecule selected from the components of the group consisting of epoxides, epihalohydrins and divinyl sulphone.

10. Gel prepared by the method according to one of claims 1 to 9.

11. Gel according to claim 10, **characterised in that** it constitutes a gel comprising at least one dispersed active ingredient.

12. Use of the gel according to claim 10 or 11 in order to separate, replace or fill a biological tissue or to increase the volume of said tissue or to supplement or replace a biological fluid.

## Patentansprüche

1. Verfahren zur Herstellung eines biologisch abbaubaren vernetzten Gels, umfassend : einen Schritt der Startens der Vernetzung einer bestimmten Menge von zumindest einem biologisch abbaubaren Polymer in Lösung durch den Zusatz einer Menge von Vernetzungsmittel,
einen Schritt der Reagierens der Vernetzung der Menge von Polymer, einen Schritt des Zusetzens einer zusätzlichen Menge von Polymer mit einer Molekularmasse von mehr als 500 000 Da in Lösung mit Verdünnung der Reaktionsmischung, um die gesamte Konzentration des Polymers in Lösung zu verringern durch Durchführen des Schritts des Vernetzens, und
einen Schritt des Stoppens der Vernetzungsreaktion durch Beseitigung des Vernetzungsmittels.

2. Verfahren nach Anspruch 1, **dadurch gekennzeichnet, dass** der Schritt des Startens der Vernetzung in einem basischen Medium durchgeführt wird.

3. Verfahren nach Anspruch 1, **dadurch gekennzeichnet, dass** der Schritt des Startens der Vernetzung in einem sauren Medium durchgeführt wird.

4. Verfahren nach einem der Ansprüche 1 bis 3, **dadurch gekennzeichnet, dass** eine zusätzlich Menge von Vernetzungsmittel beim Schritt des Zusetzens einer zusätzlichen Menge von Polymer hinzugefügt wird.

5. Verfahren nach einem der Ansprüche 1 bis 4, **dadurch gekennzeichnet, dass** der Schritt des Stoppens der Vernetzung durch Dialyse durchgeführt wird.

6. Verfahren nach einem der Ansprüche 1 bis 5, **dadurch gekennzeichnet, dass** die Polymere natürlichen Ursprungs sind.

7. Verfahren nach Anspruch 6, **dadurch gekennzeichnet, dass** die Polymere natürlichen Ursprungs Verbindungen sind, ausgewählt aus der Gruppe, die Folgendes umfasst: Hyaluronsäure, Chondroitinsulfat, Keratan, Keratansulfat, Heparin, Heparinsulfat, Zellulose und ihre Derivate, Alginate, Xanthan, Carrageen, Proteine oder Nukleinsäuren.

8. Verfahren nach Anspruch 6, **dadurch gekennzeichnet, dass** zumindest ein Polymer natürlichen Ursprungs ein Polymer ist, das im Menschen nicht natürlich vorhanden ist, ausgewählt aus der Gruppe, die Folgendes umfasst: Zellulose und ihre Derivate, Alginate, Xanthan, Carrageen, ein Polymer, das mit zumindest einem Polymer, das beim Menschen natürlich vorhanden ist, vernetzt ist, ausgewählt aus der Gruppe, die Folgendes umfasst: Hyaluronsäure, Chondroitinsulfat, Keratan, Keratansulfat, Heparin, Heparinsulfat, Proteine oder Nukleinsäuren.

9. Verfahren nach einem der Ansprüche 1 bis 8, **dadurch gekennzeichnet, dass** das Vernetzungsmittel eine bi- oder polyfunktionales Molekül ist, ausgewählt aus den Komponenten der Gruppe umfassend Epoxyde, Epihalogenhydrine und Divinylsulfon.

10. Gel, zubereitet durch das Verfahren nach einem der Ansprüche 1 bis 9.

11. Gel nach Anspruch 10, **dadurch gekennzeichnet, dass** es aus einem Gel besteht, umfassend zumindest einen dispergierten Wirkstoff.

12. Verwendung des Gels nach Anspruch 10 oder 11, um ein biologisches Gewebe zu trennen, zu ersetzen oder zu füllen oder das Volumen des Gewebes zu erhöhen oder auch ein biologisches Fluid zu ergänzen oder zu ersetzen.
